# EUROPÄISCHE PATENTSCHRIFT

(11) **EP 3 927 495 B1**
(45) Veröffentlichungstag und Bekanntmachung des Hinweises auf die Patenterteilung: **03.04.2024**
(21) Anmeldenummer: 20706455.1
(22) Anmeldetag: 19.02.2020
(51) Int. Cl.: B25J 9/00, A41D 13/05, A47C 7/38

(54) **VORRICHTUNG ZUM UNTERSTÜTZEN WENIGSTENS EINES ARMES EINES BENUTZERS UND KOPFSTÜTZE**
DEVICE FOR SUPPORTING AT LEAST ONE ARM OF A USER, AND HEADREST
DISPOSITIF POUR LE SOUTIEN D'AU MOINS UN BRAS D'UN UTILISATEUR ET APPUIE-TÊTE

(30) Priorität: 20.02.2019 DE 102019104344
(43) Veröffentlichungstag der Anmeldung: 29.12.2021
(73) Patentinhaber: Ottobock SE & Co. KGaA, 37115 Duderstadt (DE)
(72) Erfinder: LOIDL, Maximilian, 83242 Reit im Winkel (DE); MICHALKE, Lisa, 83313 Siegsdorf (DE); ZEHETMAIER, Andreas, 83530 Schnaitsee (DE); WAGNER, Robert, 83308 Trostberg (DE); MADER, Klaus, 83329 Waging (DE); ILLENSEER, Valentin, 83224 Grassau (DE); MUCK, Sebastian, 83278 Traunstein (DE); KAPELLER, Bruno, 83313 Siegsdorf (DE)
(74) Vertreter: Gramm, Lins & Partner Patent- und Rechtsanwälte PartGmbB
(86) Internationale Anmeldenummer: PCT/EP2020/054372
(87) Internationale Veröffentlichungsnummer: WO 2020/169674

(56) Entgegenhaltungen:
- DE-A1-102016 104 880
- FR-A1- 3 019 973
- US-A1- 2017 173 783
- US-B1- 6 308 345

## Beschreibung

Die Erfindung betrifft eine Vorrichtung zum Unterstützen wenigstens eines Armes eines Benutzers, wobei die Vorrichtung wenigstens ein Armstützelement mit einem Abstandselement und einer Armschale zum Anlegen an den Arm, wenigstens einen passiven Aktuator, der eingerichtet ist eine Kraft auf das Armstützelement auszuüben, und wenigstens ein Gegenlager für die aufzubringende Kraft aufweist, das wenigstens ein Kraftübertragungselement und ein Gegenlagerelement aufweist, wobei das Abstandselement schwenkbar an dem wenigstens einen Kraftübertragungselement angeordnet ist.

Eine solche Vorrichtung ist beispielsweise aus der WO 2018/224555 A1 bekannt und wird insbesondere verwendet, um den Benutzer zu unterstützen, wenn er lange Zeit die Arme heben, beispielsweise über Kopf arbeiten muss. Eine ähnliche Vorrichtung ist aus der US 2016/0081871 A1 bekannt. Sie verfügt über ein Gegenlagerelement, das in Form eines um den Rumpf des Benutzers herumlegbaren Gurtes ausgebildet ist, an dem sich zwei entlang des Rückens zur Schulter verlaufende Stützstreben befinden, die oberhalb und seitlich neben den Schultern des Benutzers mit jeweils einem Gelenk verbunden sind, so dass der Arm angehoben werden kann. Federelemente, die an den Gelenken positioniert sind, üben eine nach oben gerichtete Kraft auf die Armschalen aus, so dass beispielsweise beim Heben schwerer Gegenstände eine Unterstützung der Arme erfolgt. Sollen die Arme gesenkt werden, muss durch die Arme ein Druck auf die Armschalen ausgeübt werden, der die von den Federelementen aufgebrachte Kraft übersteigt.

Insbesondere beim Arbeiten über Kopf aber auch in anderen Situationen, in denen lange die Arme eines Benutzers angehoben werden müssen, leisten derartige Vorrichtungen gute Dienste beim Unterstützen der Arme. Herkömmlicherweise wird jedoch der Benutzer mit seinem Blick den Händen folgen, um zu sehen und zu beobachten, welche Arbeiten ausgeführt und was als nächstes zu tun ist. Dies bedeutet, der Kopf des Benutzers wird in den Nacken gelegt, um den Blick nach oben wenden zu können. Insbesondere wenn diese Stellung lange eingenommen werden muss, kann es zu Verspannungen im Nackenbereich und im hinteren Kopfbereich kommen, die unangenehm und schmerzhaft sein können, und weitere Haltungsschäden und Beeinträchtigungen des Benutzers zur Folge haben können.

Aus der US 6,308,345 B1 ist eine Kopfstütze bekannt, die eine Stützplatte und einen damit einstückig verbundenen Stützanteil aufweist. Die Stützplatte wird am Rücken im Bereich der Schulterblätter angeordnet, sodass der Stützanteil am Kopf anliegt. Nachteilig ist, dass die Vorrichtung aufwendig anzulegen ist, da die Platte über ein Geschirr aus mehreren Riemen und Gurten am Oberkörper befestigt werden muss. Die US 2017/0173783 A1 hingegen zeigt eine Vorrichtung, die eingerichtet ist, eine Kraft auf die Oberarme eines Benutzers auszuüben, wenn dieser beispielsweise über Kopf arbeiten muss. Die Vorrichtung, die über eine massive entlang der Wirbelsäule angeordnete Stange verfügt, die mittels Schultergurt und einem Hüftgurt am Oberkörper befestigt wird, verfügt über ausladende Stützelemente, an denen der eigentliche Aktuator befestigt ist, der die Kraft auf die Oberarme aufbringt. Die Vorrichtung verfügt auch über ein am oberen Ende der Stange befestigtes Kopfstützelement.

Der Erfindung liegt daher die Aufgabe zugrunde, eine Vorrichtung gemäß dem Oberbegriff des Anspruchs 1 so weiterzuentwickeln, dass sie diese Nachteile beseitigt.

Die Erfindung löst die gestellte Aufgabe durch eine Vorrichtung gemäß Anspruch 1. Die Vorrichtung weist eine Kopfstütze auf, die wenigstens ein Schulterelement und wenigstens ein Stützelement aufweist, das lösbar an dem wenigstens einen Schulterelement angeordnet und elastisch ist, wobei die Kopfstütze derart ausgebildet ist, das sie im angelegten Zustand der Vorrichtung den Kopf des Benutzers stützt, wenn der Kopf wenigstens um einen vorbestimmten Grenzwinkel nach hinten geneigt wird, wobei das Schulterelement mehrere Bügle aufweist, von denen wenigstens einer über jede der beiden Schultern des Benutzers verläuft.

Die Kopfstütze der Vorrichtung verfügt erfindungsgemäß folglich über wenigstens ein Schulterelement, das an der Schulter des Benutzers angeordnet wird. Dabei handelt es sich erfindungsgemäß um mehrere Bügel, die über die Schulter gelegt werden und im Wesentlichen wie ein nach unten geöffnetes U ausgebildet sind. Erfindungsgemäß verläuft wenigstens ein Bügel über jede der beiden Schultern des Benutzers, wobei diese beide Bügel vorteilhafterweise ventral, also vorn, und/oder dorsal, also hinten, miteinander verbunden sind. In einer besonders bevorzugten Ausgestaltung handelt es sich bei dem wenigstens einen Schulterelement um einen Rahmen, der um den Kopf herum verläuft und an den Schultern zwei nach unten geöffnete U-förmige Elemente aufweist, die sowohl ventral als auch dorsal miteinander verbunden sind.

An dem wenigstens einen Schulterelement ist das wenigstens eine Stützelement erfindungsgemäß lösbar angeordnet ist. Dadurch wird erreicht, dass beispielsweise das Stützelement leicht vom Schulterelement entfernt und beispielsweise durch ein anderes Stützelement ausgetauscht werden kann, das in einer bevorzugten Ausgestaltung andere geometrische Abmessungen ausweist. Auf diese Weise können unterschiedliche Schulterelemente und unterschiedliche Stützelemente miteinander kombiniert werden, um so die für den jeweiligen Benutzer passenden Abmessungen von Schulterelement und Stützelement miteinander zu kombinieren. Dies geschieht beispielsweise durch Formschlusselemente, die zueinander korrespondierend sowohl an dem wenigstens einen Schulterelement als auch an dem wenigstens einen Stützelement angeordnet sind. Derartige Formschlusselemente können beispielsweise Klettverschlusselemente, Druckknöpfe, Schnapp- oder Clipelemente oder sonstige Formschlusselemente sein.

Bevorzugt erstreckt sich das Stützelement dorsal, also hinten, von dem wenigstens einen Schulterelement nach oben Richtung Kopf. Es ist erfindungsgemäß elastisch ausgebildet. Die Elastizität bezieht sich dabei insbesondere darauf, dass das Stütz-element für den Kopf keinen festen und unverrückbaren Anschlag bildet, an dem der Kopf beim nach hinten neigen anschlägt. Dies kann schmerzhaft, zumindest jedoch unangenehm sein und zudem dazu führen, dass der Kopf zwar gestützt wird, dies jedoch in einer vorher festgesetzten Position, die gegebenenfalls nicht der gewünschten Position entspricht. Ist das wenigstens eine Stützelement elastisch ausgebildet, kann es dem von dem Kopf auf das Stützelement aufgebrachten Druck zumindest in einem vorbestimmten Bereich nachgeben und so die optimale Stützposition für die jeweils gewünschte Arbeitsposition der Hände oder Blickrichtung des Kopfes ermöglichen.

In einer bevorzugten Ausgestaltung verfügt die Vorrichtung über mehrere unterschiedlich ausgebildete Stützelemente, die beispielsweise unterschiedlich lang ausgebildet sein können, so dass sie sich unterschiedlich weit von dem wenigstens einen Element nach oben erstrecken, wenn sie mit dem wenigstens einen Schulterelement verbunden sind. Auf diese Weise kann der Grenzwinkel eingestellt und verändert werden, bei dem das Stützelement mit dem nach hinten geneigten Kopf in Kontakt kommt. Bevorzugt liegt der Grenzwinkel zwischen 5° und 45°, besonders bevorzugt zwischen 10° und 30°, weiter bevorzugt zwischen 15° und 25°, höchst bevorzugt bei 20°. Der Winkel bezeichnet dabei den Winkel, um den der Kopf des Benutzers der Vorrichtung aus der aufrechten Position nach hinten geneigt werden muss, um mit dem Stützelement oder einem anderen Bauteil der Kopfstütze in Kontakt zu kommen. Bevorzugt erlaubt die Elastizität der Kopfstütze ab diesem Moment, also dem Erreichen des Grenzwinkels eine weitere Neigung des Kopfes um wenigstens 5°, bevorzugt wenigstens 10°, besonders bevorzugt um wenigstens 15° und um höchstens 30°, bevorzugt höchstens 25°, besonders bevorzugt höchstens 20°.

In einer bevorzugten Ausgestaltung ermöglicht das Stützelement dem Benutzer eine Drehung des Kopfes auch in dem Zustand, in dem der Kopf an dem Stützelement anliegt. Dabei kann das Stützelement so ausgebildet sein, dass es an der Rückseite des Kopfes beim Drehen des Kopfes entlanggleitet, ohne dass die Position, an der das Stützelement an dem Kopf anliegt, fest und konstant bleibt, und das Stützelement aufgrund seiner Elastizität eine Bewegung des Kopfes erlaubt, so dass es sich dabei verformt.

Vorteilhafterweise ist das wenigstens eine Stützelement in mehreren Positionen, vorzugsweise stufenlos verstellbar, an dem wenigstens einen Schulterelement befestigbar. Auf diese Weise kann den individuellen Abmessungen und Größenverhältnissen des jeweiligen Benutzers Rechnung getragen werden. Dies ist besonders einfach dann möglich, wenn das wenigstens eine Stützelement an dem wenigstens einen Schulterelement mittels Klettverschlüssen angeordnet ist. Auf diese Weise ist insbesondere die stufenlose Verstellbarkeit leicht zu erreichen.

In einer bevorzugten Ausgestaltung befindet sich an dem wenigstens einen Stützelement wenigstens ein Kopfanlageelement zum Anlegen an den Kopf des Benutzers, das sich vorzugsweise an eine Kontur des Kopfes anpasst, wenn es mit dem Kopf des Benutzers in Kontakt kommt. Ein solches Kopfanlageelement kann beispielsweise als Kissen ausgebildet und vorzugsweise aus einem Schaumstoff hergestellt sein, der besonders bevorzugt mit einem Stoffüberzug aus einem textilen Material überzogen ist. Besonders bevorzugt ist ein solches Kopfanlageelement lösbar an dem Stützelement angeordnet, um beispielsweise zu Reinigungs- oder Reparaturzwecken oder beispielsweise beim Wechsel des Benutzers entfernt und durch ein anders Kopfanlageelement ersetzt zu werden.

Besonders bevorzugt verfügt das wenigstens eine Stützelement über wenigstens zwei Kopfanlageelemente, die mit dem Kopf des Benutzers, vorzugsweise im Bereich der Linea nuchalis superior, also der oberen Nackenlinie, der Linea nuchalis inferior, also der unteren Nackenlinie, besonders bevorzugt seitlich der Protuberantia occipitalis externa, also dem äußeren Hirnhauptvorsprung, anliegen.

Besonders bevorzugt weist das wenigstens eine Kopfanlageelement ein flächiges, flexibles, vorzugsweise elastisches Material auf oder besteht daraus und ist bevorzugt derart an dem Stützelement angeordnet, dass sich zwischen dem Kopfanlageelement und dem Stützelement ein Spalt befindet, der im bestimmungsgemäßen Gebrauch nicht geschlossen wird. Diese Konstruktion kann auch als "Hängematte" angesehen und bezeichnet werden, bei dem das eigentliche Kopfanlageelement die Hängematte und das Stützelement die entsprechende Stützkonstruktion bildet. Das Kopfanlageelement ist am Rand an zwei oder mehr Stellen, gegebenenfalls auch am vollständigen umlaufenden Rand, mit dem wenigstens einen Stützelement so verbunden, dass es zwischen den Befestigungsstellen aufgespannt wird oder zumindest so angeordnet ist, dass es zwischen den Anlagenstellen nicht mit dem Stützelement in Kontakt kommt. Wird nun der Kopf des Benutzers um wenigstens den vorbestimmten Grenzwinkel nach hinten geneigt, kommt er in Kontakt mit dem Kopfanlageelement, das sich aufgrund der Hängemattenstruktur und -anordnung optimal an die Kopfform des Benutzers anpassen kann. Dies ist unabhängig davon, ob der Kopf gerade nach hinten geneigt wird, also die Blickrichtung weiterhin nach vorne zeigt, oder ob der Kopf zusätzlich zu der Neigung nach hinten nach rechts oder links gedreht wird. Die Anpassung erfolgt durch die Konstruktion als Hängematte immer in optimaler Weise. Dadurch, dass der Spalt zwischen dem wenigstens einen Kopfanlageelement und dem wenigstens einen Stützelement durch die im bestimmungsgemäßen Gebrauch auftretenden Kräfte nicht geschlossen wird, wird auch eine gute Polsterung des Kopfes erreicht, da dieser auch im abgestützten Zustand nicht mit dem eigentlichen Stützelement in Kontakt kommt.

Vorteilhafterweise verfügt die Kopfstütze über wenigstens einen Gurt, bevorzugt wenigstens zwei Gurte, die an dem Gegenlager, bevorzugt an dem Gegenlagerelement befestigbar oder befestigt sind. Dadurch wird die Position der Kopfstütze auch im belasteten Zustand, also mit nach hinten geneigtem Kopf des Benutzers, am Körper des Benutzers gesichert. Der wenigstens eine Gurt wird beispielsweise über Formschlusselemente, beispielsweise Clips- oder Schnappelemente oder durch Schnallenelemente an dem Gegenlager, bevorzugt dem Gegenlagerelement, insbesondere einem Hüftgurt, positioniert.

In einer bevorzugten Ausgestaltung verläuft dabei der wenigstens eine Gurt im angelegten Zustand der Vorrichtung ventral, also vorn, am Körper des Benutzers entlang. Diese Ausgestaltung ist insbesondere deswegen von Vorteil, weil durch den nach hinten geneigten Kopf eine nach hinten wirkende Kraft auf die Nackenstütze ausgeübt wird, die durch vorn am Körper entlanglaufende Gurte besonders gut, einfach und sicher auf das Gegenlagerelement, vorzugsweise den Hüftgurt übertragen werden kann. Zudem ist die Vorrichtung und insbesondere die Kopfstütze mit dieser Ausgestaltung des wenigstens einen Gurtes auf besonders einfache Weise anlegbar, da der wenigstens eine Gurt vorn am Körper am Gegenlagerelement befestigt werden muss.

Alternativ oder zusätzlich dazu kann der wenigstens eine Gurt im angelegten Zustand der Vorrichtung auch dorsal am Körper des Benutzers verlaufen. Dies ist insbesondere dann von Vorteil, wenn beispielsweise durch das wenigstens eine Schulterelement eine ausreichende Stabilität der Position der Kopfstütze am Körper des Benutzers gegeben ist.

Vorzugsweise verfügt wenigstens einer der Gurte über ein Formschlusselement, insbesondere ein Schnappelement und/oder ein Clipselement, wobei der Gurt an dem Gegenlager befestigbar ist, indem das Formschlusselement mit einem korrespondierenden Gegenelement zusammenwirkt.

Vorteilhafterweise ist die Elastizität des Stützelementes einstellbar. Dies kann beispielsweise durch zusätzliche Verstärkungselemente erreicht werden, die an einer elastischen Strebe oder einem elastischen Teil des Stützelementes angeordnet werden können und auf diese Weise beispielsweise die Wandstärke erhöhen, so dass die Elastizität abnimmt. Alternativ oder zusätzlich dazu kann auch das wenigstens eine Stützelement selbst aus mehreren Bestandteilen bestehen, die in unterschiedlichen Orientierungen oder mit unterschiedlich größer Kontaktfläche zueinander positioniert werden können. Auch dadurch kann die Elastizität eingestellt werden.

Vorteilhafterweise ist das wenigstens eine Schulterelement elastisch ausgebildet. Dies ist beispielsweise dann von Vorteil, wenn es eine geometrische Form aufweist, die durch das Aufsetzen auf die Schulter des Benutzers aufgeweitet wird. Dadurch kommt es zu einer Klemmwirkung, die ein Verrutschen des Schulterelementes relativ zum Körper des Benutzers zumindest erschwert, bevorzugt ganz verhindert.

Die Erfindung löst die gestellte Aufgabe zudem durch eine Kopfstütze gemäß Anspruch 11. Die Kopfstütze weist wenigstens ein Schulterelement und wenigstens ein Stützelement auf, das lösbar an dem wenigstens einen Schulterelement angeordnet und elastisch ist, wobei die Kopfstütze derart ausgebildet ist, das sie im angelegten Zustand der Vorrichtung den Kopf des Benutzers stützt, wenn der Kopf wenigstens um einen vorbestimmten Grenzwinkel nach hinten geneigt wird. Eine derartige Kopfstütze ist insbesondere für die Vorrichtung der hier beschriebenen Art geeignet.

Vorzugsweise verfügt die Kopfstütze über wenigstens einen Gurt, der wenigstens ein Befestigungselement, insbesondere wenigstens ein Formschlusselement, aufweist, durch das der wenigstens eine Gurt an einem Gegenlager, das vorteilhafterweise ein Hüftgurt ist, befestigbar ist, und/oder dadurch, dass das wenigstens eine Schulterelement nicht aus einem flexiblen Material hergestellt ist. Anderenfalls könnte das Schulterelement beispielsweise auch als Gurt oder Gurtpaar ausgebildet sein.

Mit Hilfe der beiliegenden Figuren werden nachfolgend einige Ausführungsbeispiele der vorliegenden Erfindung näher erläutert. Es zeigen:
- Figuren 1 bis 4 -: unterschiedliche Darstellungen einer Kopfstütze gemäß einem ersten Ausführungsbeispiel der vorliegenden Erfindung,
- Figuren 5 bis 8 -: die Darstellungen aus den Figuren 1 bis 4 für ein zweites Ausführungsbeispiel der vorliegenden Erfindung,
- Figuren 9 und 10 -: zwei Darstellungen einer Kopfstütze gemäß einem weiteren Ausführungsbeispiel der vorliegenden Erfindung,
- Figuren 11 und 12 -: zwei Darstellungen einer Kopfstütze gemäß einem weiteren Ausführungsbeispiel,
- Figuren 13 bis 15 -: Detaildarstellungen einer Kopfstütze gemäß einem weiteren Ausführungsbeispiel der vorliegenden Erfindung und
- Figuren 16 und 17-: zwei Darstellungen einer Vorrichtung gemäß einem Ausführungsbeispiel der vorliegenden Erfindung.

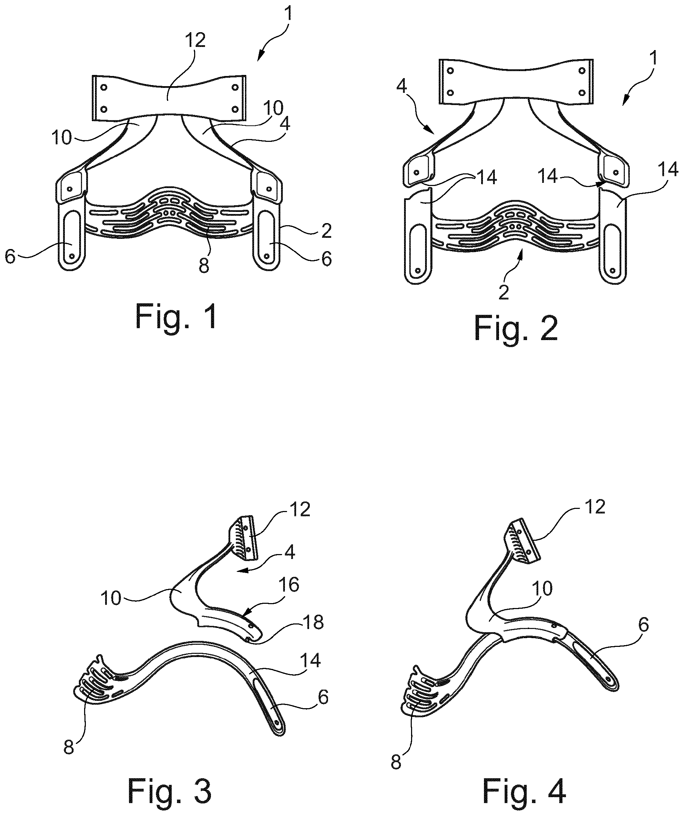

Figur 1 zeigt eine Kopfstütze 1, die ein Schulterelement 2 und ein Stützelement 4 aufweist. Das Schulterelement 2 verfügt über zwei Schulterbügel 6, die im angelegten Zustand der Kopfstütze 1 über die Schultern des Benutzers gelegt werden. Sie sind dorsal, also im Bereich des Rückens durch eine Verbindungsstrebe 8 miteinander verbunden. An dem Schulterelement 2 befindet sich das Stützelement 4, das im gezeigten Ausführungsbeispiel zwei Stützbügel 10 aufweist, von denen sich jeweils einer von jeweils einem der beiden Schulterbügel 6 nach oben erstreckt. Sie sind im oberen Bereich durch ein Kopfanlageelement 12 miteinander verbunden, an dem der Kopf des Benutzers anliegt.

Figur 2 zeigt die Darstellung aus Figur 1 mit getrennten Bauteilen. Das Schulterelement 2 ist von dem Stützelement 4 getrennt. Beide verfügen über Klettverschlusselemente 14, durch die die beiden Bauteile in unterschiedlichen Positionen stufenlos verstellbar aneinander befestigt werden können.

Figur 3 zeigt die Darstellung aus Figur 2 in einer Seitenansicht. Man erkennt einen der Schulterbügel 6, an denen sich das Klettverschlusselement 14 befindet. Es ist im hinteren Bereich durch die Verbindungsstrebe 8 mit dem in Figur 3 nicht gezeigten zweiten Schulterbügel 6 verbunden. Das Stützelement 4, an dem das Kopfanlageelement 12 angeordnet ist, verfügt über die beiden Stützbügel 10, von denen ebenfalls nur einer dargestellt ist. In einem Kontaktbereich 16, in dem der Stützbügel 10 mit dem jeweiligen Schulterbügel 6 in Kontakt kommt, verfügt der Stützbügel 10 über eine Schiene 18, die eingerichtet ist, den jeweiligen Schulterbügel 6 zumindest teilweise zu umfassen. Dennoch befinden sich die Klettverschlusselemente 14 in Position, um die Haltekraft zwischen dem Stützelement 4 und dem Schulterelement 2 zu vergrößern. Figur 4 zeigt die Darstellung aus Figur 1 in einer Seitenansicht. Man erkennt wieder einen der Schulterbügel 6, einen der Stützbügel 10 mit dem daran angeordneten Kopfanlageelement 12 sowie der Verbindungsstrebe 8, die die beiden Schulterbügel 6 miteinander verbindet.

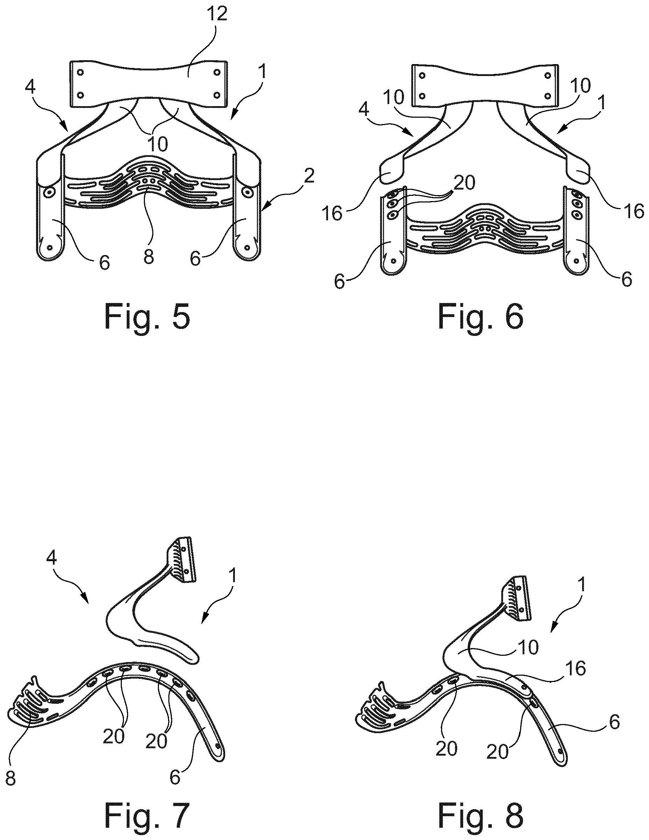

Die Figuren 5 bis 8 zeigen ein weiteres Ausführungsbeispiel der Kopfstütze 1. Auch sie verfügt über das Schulterelement 2 mit den beiden Schulterbügeln 6, die durch die Verbindungsstrebe 8 miteinander verbunden sind. Das Stützelement 4 zeigt wieder die beiden Stützbügel 10, an denen sich im oberen Bereich das Kopfanlageelement 12 befindet. Im Gegensatz zu der Ausführungsform aus den Figuren 1 bis 4 ist hier jedoch das Stützelement 4 am Schulterelement 2 über Druccknöpfe angeordnet, was insbesondere in Figur 6 dargestellt ist. Man erkennt die entsprechenden Formschlusselemente 20, die an den Schulterbügeln 6 positioniert sind und mit Gegenelementen zusammenwirken können, die an der Unterseite des Kontaktbereiches 16 der jeweiligen Stützbügels 10 angeordnet, in den Figuren jedoch nicht dargestellt sind. Auch auf diese Weise lässt sich die Position des Schulterelementes 2 relativ zum Stützelement 4 verändern, dies ist jedoch anders als bei der Klettverschlussvariante nicht stufenlos der Fall.

Die Figuren 7 und 8 zeigen die Seitendarstellung. Der Schulterbügel 6 mit der Verbindungsstrebe 8 ist in Figur 7 losgelöst von dem Stützelement 4 dargestellt. Man erkennt deutlich die Formschlusselemente 20, die an dem Schulterbügel 6 angeordnet sind. Figur 8 zeigt die Darstellung in der verbundenen Position. Dabei sind noch immer einige der Formschlusselemente sichtbar, während der Kontaktbereich 16 des Stützbügels 10 mit dem Schulterbügel 6 in Kontakt gebracht wurde.

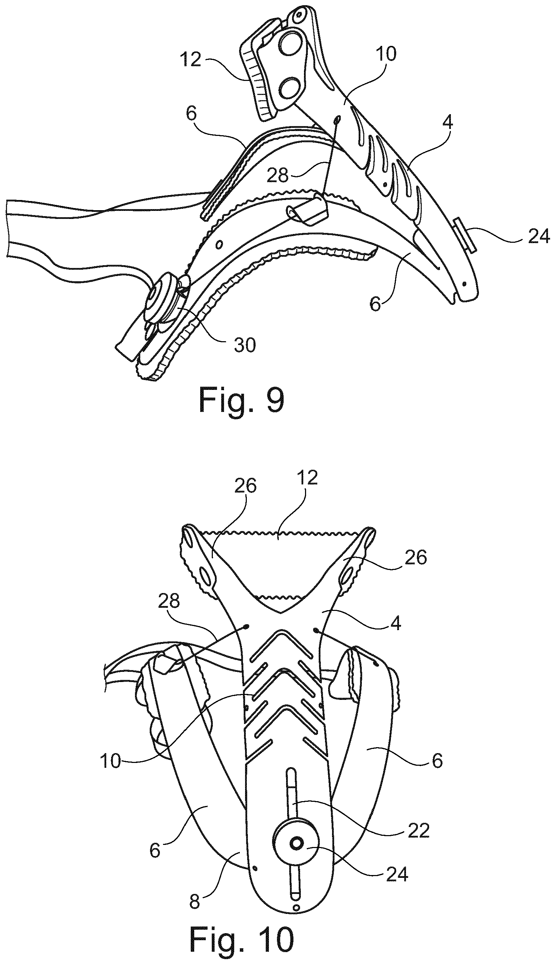

Die Figuren 9 und 10 zeigen eine andere Ausführungsform. Sie verfügt über zwei Schulterbügel 6, die aus einem festen jedoch elastischen Kunststoff bestehen und über die Schultern des Trägers gelegt werden. Sie sind im Rückenbereich über eine kurze Verbindungsstrebe 8 miteinander verbunden, an der sich das Stützelement 4 befindet. Im Stützelement 4 befindet sich ein Langloch 22, durch das eine Schraube 24 hindurchgeführt ist, die mit der Verbindungsstrebe 8 der beiden Schulterbügel 6 verbunden ist. In der Verbindungsstrebe 8 befindet sich eine Bohrung mit einem Innengewinde, in das die Schraube 24 eingreift. Wird die Schraube 24 gelöst, kann das Stützelement 4 entlang des Langloches 22 relativ zu den Schulterbügeln 6 und damit relativ zum Schulterelement 2 verschoben werden. Ist die gewünschte Position erreicht, wird die Schraube 24 angezogen und eine weitere Verschiebung ist nicht möglich.

Das Stützelement 4 verfügt nur über einen Stützbügel 10, der über eine Reihe von Ausnehmungen und Durchbrüchen verfügt, durch die die Elastizität des Stützbügels 10 erreicht wird. Am oberen Ende des Stützbügels 10 verfügt er über zwei Vorsprünge 26, die sich U-förmig oder V-förmig voneinander entfernen. Zwischen den beiden ist das eigentliche Kopfanlageelement hier als textiles Element ausgebildet, angeordnet.

Die Ausführungsform in den Figuren 9 und 10 verfügt zudem über eine Spannvorrichtung 28, die in Form eines Drahtes oder Bandes ausgebildet ist, das durch ein entsprechendes Spannelement 30 aufgewickelt oder abgewickelt werden kann. Dadurch wird die wirksame Länge der Spannvorrichtung verändert, so dass das obere Ende des Stützbügels 10 nach unten gezogen oder wieder nach oben entlassen werden kann. Dadurch wird die Position, an der das Kopfanlageelement 12 mit einem Kopf in Kontakt kommt, verändert.

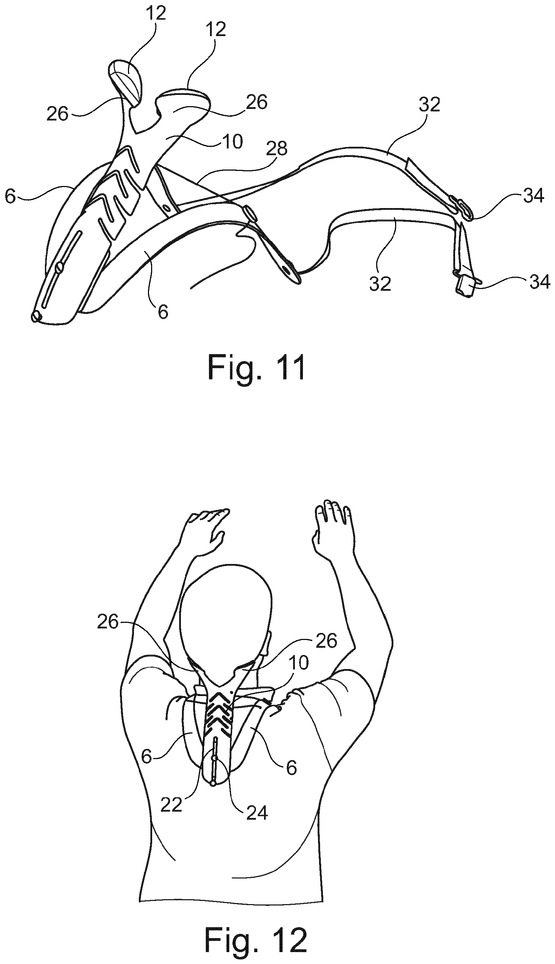

Die Figuren 11 und 12 zeigen eine weitere Ausführungsform, wobei Figur 12 sie im angelegten Zustand zeigt. Auch sie verfügt über zwei Schulterbügel 6, die im dorsalen Bereich miteinander verbunden sind. In diesem Bereich ist wieder der einzige Stützbügel 10 angeordnet, der über ein Langloch 22 verfügt, in dem eine Schraube 24 angeordnet ist. Auch dieser Stützbügel 10 verfügt über eine Reihe von Ausnehmungen und Bohrungen, durch die die Elastizität des an sich schon elastischen Kunststoffmaterials weiter vergrößert wird.

Im oberen Bereich des Stützbügels 10 spaltet auch dieser sich in zwei Vorsprünge 26 auf, an denen nun anders als in den Figuren 9 und 10 jedoch zwei Kopfanlageelemente 12 angeordnet sind. Figur 12 zeigt die in Figur 8 gezeigte Vorrichtung im angelegten Zustand, bei der zu erkennen ist, dass die Kopfanlageelemente 26 im Bereich des hinteren Schädels an den Kopf anliegen.

In Figur 11 ist zu erkennen, dass auch diese Kopfstütze 1 über eine Spannvorrichtung 28 verfügt, die den bezüglich der Figuren 9 und 10 bereits beschriebenen Effekt hat. Zusätzlich verfügt die Vorrichtung über zwei Gurte 32, an deren der Kopfstütze abgewandten Ende sich ein Befestigungselement 34 befindet. Im gezeigten Ausführungsbeispiel handelt es sich um eine Klemmschnalle, wie sie beispielsweise von Hosenträgern bekannt ist. Durch diese Befestigungselemente 34 können die Gurte 32 und damit die gesamte Kopfstütze 1 an einem Gegenlagerelement, beispielsweise an einem Hüftgurt oder Hosengürtel befestigt werden.

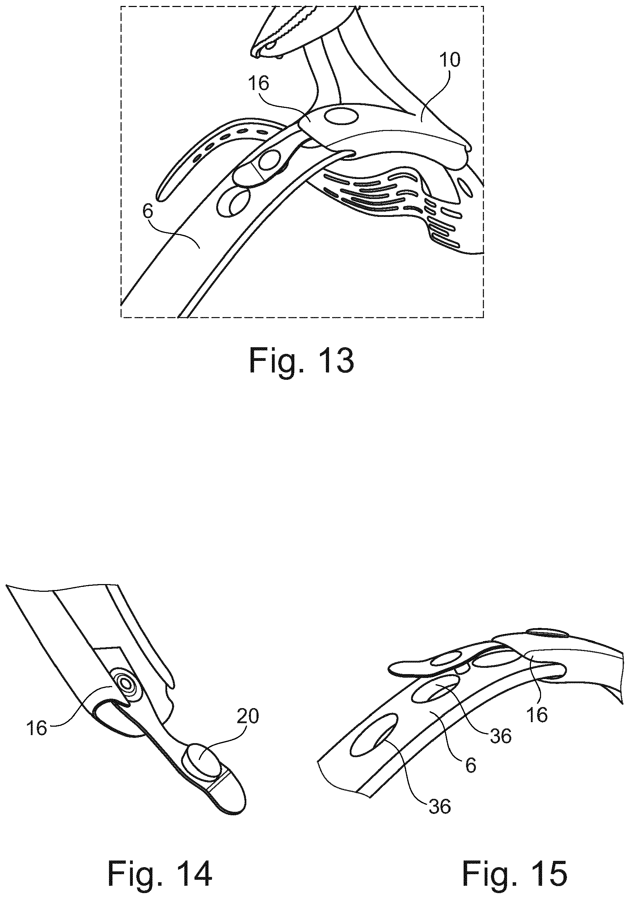

Die Figuren 13 bis 15 zeigen Details der Verbindungseinrichtung zwischen Schulterbügel 6 und dem Kontaktbereich 16 der Stützbügel 10. Die Schulterbügel 6 verfügen über Ausnehmungen 36, durch die entsprechende Formschlusselemente geführt werden können, die am vorderen Ende des Kontaktbereiches 16 der Stützbügel 10 angeordnet sind. Ein solches Formschlusselement 20 ist insbesondere in Figur 14 dargestellt. Auf diese Weise lässt sich der Stützbügel 10 in unterschiedlichen Positionen am Schulterbügel 6 befestigen.

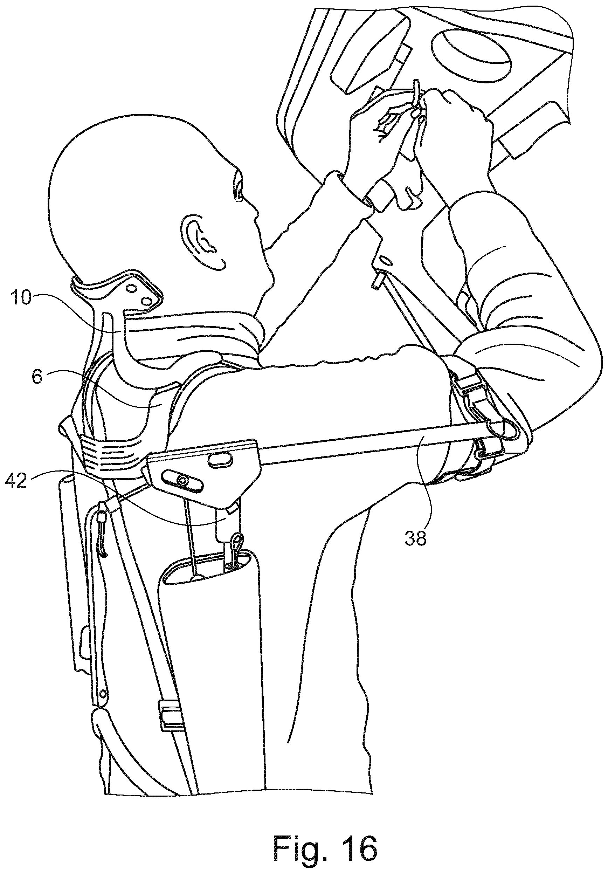

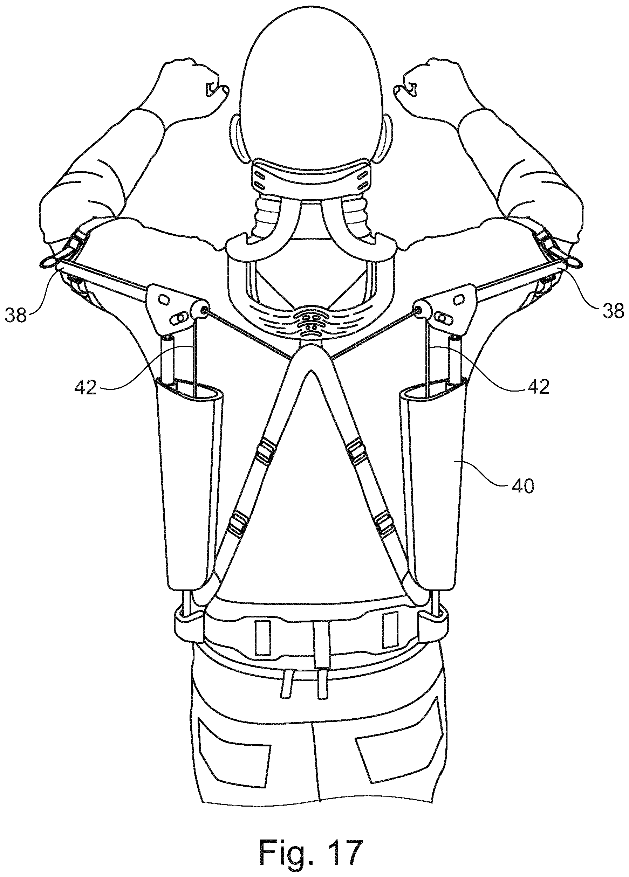

Figuren 16 und 17 zeigen eine Vorrichtung zum Unterstützen zweier Arme im angelegten Zustand. Sie verfügt über zwei Armstützelemente 38, die mit jeweils einem Kraftübertragungselement 40 verbunden sind. Sie verfügen über jeweils einen passiven Aktuator 42 und über ein Gegenlagerelement in Form eines Hüftgurtes.

Teil der Vorrichtung ist zudem die Kopfstütze mit dem Schulterelement 2, deren beiden Schulterbügeln 6 sowie dem Stützelement 4 mit den Stützbügeln 10.

### Bezugszeichenliste

- 1: Kopfstütze
- 2: Schulterelement
- 4: Stützelement
- 6: Schulterbügel
- 8: Verbindungsstrebe
- 10: Stützbügel
- 12: Kopfanlageelement
- 14: Klettverschlusselement
- 16: Kontaktbereich
- 18: Schiene
- 20: Formschlusselement
- 22: Langloch
- 24: Schraube
- 26: Vorsprung
- 28: Spannvorrichtung
- 30: Spannelement
- 32: Gurt
- 34: Befestigungselement
- 36: Ausnehmung
- 38: Armstützelement
- 40: Kraftübertragungselement
- 42: passiver Aktuator

## Patentansprüche

1. Vorrichtung zum Unterstützen wenigstens eines Armes eines Benutzers, wobei die Vorrichtung
a. wenigstens ein Armstützelement (38) mit einem Abstandselement und einer Armschale zum Anlegen an den Arm,
b. wenigstens einen passiven Aktuator (42),
i. der eingerichtet ist, eine Kraft auf das Armstützelement (38) auszuüben, und
c. wenigstens ein Gegenlager für die aufzubringende Kraft aufweist,
i. das wenigstens ein Kraftübertragungselement (40) und ein Gegenlagerelement aufweist,
wobei das Abstandselement schwenkbar an dem wenigstens einen Kraftübertragungselement (40) angeordnet ist,
wobei die Vorrichtung eine Kopfstütze (1) aufweist, die wenigstens ein Schulterelement (2) und wenigstens ein Stützelement (4) aufweist, das lösbar an dem wenigstens ein Schulterelement (2) angeordnet und elastisch ist,
wobei die Kopfstütze (1) derart ausgebildet ist, dass sie im angelegten Zustand der Vorrichtung den Kopf des Benutzers stützt, wenn der Kopf wenigstens um einen vorbestimmten Grenzwinkel nach hinten geneigt wird,
wobei das Schulterelement (2) mehrere Bügel (6) aufweist, von denen jeweils wenigstens einer über jede der beiden Schultern des Benutzers verläuft.

2. Vorrichtung nach Anspruch 1, **dadurch gekennzeichnet, dass** das wenigstens eine Stützelement (4) in mehreren Positionen, vorzugsweise stufenlos verstellbar, an dem wenigstens einen Schulterelement (2) befestigbar ist.

3. Vorrichtung nach Anspruch 1 oder 2, **dadurch gekennzeichnet, dass** an dem wenigstens einen Stützelement (4) wenigstens ein Kopfanlageelement (12) zum Anlegen an den Kopf des Benutzers angeordnet ist, das sich vorzugsweise an eine Kontur des Kopfes anpasst, wenn es mit dem Kopf des Benutzers in Kontakt kommt.

4. Vorrichtung nach Anspruch 3, **dadurch gekennzeichnet, dass** das wenigstens eine Kopfanlageelement (12) ein flächiges, flexibles, vorzugsweise elastisches Material aufweist oder daraus besteht und derart an dem Stützelement (4) angeordnet ist, dass sich zwischen dem Kopfanlageelement (12) und dem Stützelement (4) ein Spalt befindet, der im bestimmungsgemäßen Gebrauch nicht geschlossen wird.

5. Vorrichtung nach einem der vorstehenden Ansprüche, **dadurch gekennzeichnet, dass** die Kopfstütze (1) wenigstens einen Gurt (32), bevorzugt wenigstens zwei Gurte (32) aufweist, die an dem Gegenlager, bevorzugt an dem Gegenlagerelement befestigbar oder befestigt sind.

6. Vorrichtung nach Anspruch 5, **dadurch gekennzeichnet, dass** wenigstens ein Gurt (32) im angelegten Zustand der Vorrichtung ventral am Körper des Benutzers verläuft.

7. Vorrichtung nach Anspruch 5 oder 6, **dadurch gekennzeichnet, dass** wenigstens ein Gurt (32) im angelegten Zustand der Vorrichtung dorsal am Körper des Benutzers verläuft.

8. Vorrichtung nach einem der Ansprüche 5, 6 oder 7, **dadurch gekennzeichnet, dass** wenigstens einer der Gurte (32) ein Formschlusselement (20), insbesondere ein Schnappelement und/oder ein Clipselement, aufweist, wobei der Gurt (32) an dem Gegenlager befestigbar ist, indem das Formschlusselement 20() mit einem korrespondierenden Gegenelement zusammenwirkt.

9. Vorrichtung nach einem der vorstehenden Ansprüche, **dadurch gekennzeichnet, dass** die Elastizität des Stützelementes (4) einstellbar ist.

10. Vorrichtung nach einem der vorstehenden Ansprüche, **dadurch gekennzeichnet, dass** das wenigstens eine Schulterelement (2) elastisch ausgebildet ist.

11. Kopfstütze (1) insbesondere für eine Vorrichtung nach einem der vorstehenden Ansprüche, wobei die Kopfstütze (1) wenigstens ein Schulterelement (2) und wenigstens ein Stützelement (4) aufweist, das lösbar an dem wenigstens ein Schulterelement (2) angeordnet und elastisch ist, wobei die Kopfstütze (1) derart ausgebildet ist, dass sie im angelegten Zustand der Vorrichtung den Kopf des Benutzers stützt, wenn der Kopf wenigstens um einen vorbestimmten Grenzwinkel nach hinten geneigt wird, wobei das Schulterelement (2) mehrere Bügel aufweist, von denen jeweils wenigstens einer über jede der beiden Schultern des Benutzers verläuft.

12. Kopfstütze nach Anspruch 11, **dadurch gekennzeichnet, dass** die Kopfstütze wenigstens einen Gurt (32) aufweist, der wenigstens ein Befestigungselement (34) aufweist, durch das der wenigstens eine Gurt (32) an einem Gegenlager, vorzugsweise einem Hüftgurt, befestigbar ist, und/oder dadurch, dass das wenigstens eine Schulterelement (2) nicht aus einem flexiblen Material hergestellt ist.

## Claims

1. A device for supporting at least one arm of a user, wherein the device has
a. at least one arm support element (38) with a spacer element and an arm shell for mounting on the upper arm,
b. at least one passive actuator (42),
i. which is configured to apply a force to the arm support element (38), and
c. at least one counter bearing for the force to be applied,
i. which comprises at least one force transmission element (40) and a counter bearing element,
wherein the spacer element is arranged on the at least one force transmission element (40) such that it can be swivelled,
wherein the device has a headrest (1) with at least one shoulder element (2) and at least one support element (4) that is arranged on the at least one shoulder element (2) such that it can be detached and is elastic,
wherein the headrest (1) is designed in such a way that, when the device is mounted, it supports the user's head when the head is tilted backwards at least by a predetermined limit angle,
wherein the shoulder element (2) comprises multiple brackets (6), at least one of which extends respectively across each of the two shoulders of the user.

2. The device according to claim 1, **characterized in that** the at least one support element (4) can be fixed on the at least one shoulder element (2) in multiple positions, which are preferably infinitely adjustable.

3. The device according to claim 1 or 2, **characterized in that** at least one head support element (12) for resting against the user's head is arranged on the at least one support element (4), said head support element (12) preferably adapting to a contour of the head when it comes into contact with the user's head.

4. The device according to claim 3, **characterized in that** the at least one head support element (12) features a flat, flexible, preferably elastic material, or is composed of such a material, and is arranged on the support element (4) in such a way that there is a gap between the head support element (12) and the support element (4) that is not closed when the device is used as intended.

5. The device according to one of the preceding claims, **characterized in that** the headrest (1) comprises at least one strap (32), preferably at least two straps (32), that can be or are fixed to the counter bearing, preferably to the counter bearing element.

6. The device according to claim 5, **characterized in that** the at least one strap (32) extends ventrally on the user's body when the device is mounted.

7. The device according to claim 5 or 6, **characterized in that** the at least one strap (32) extends dorsally on the user's body when the device is mounted.

8. The device according to one of the claims 5, 6 or 7, **characterized in that** at least one of the straps (32) comprises a positive-locking element (20), in particular a snap element and/or a clip element, wherein the strap (32) can be fixed to the counter bearing by engaging the positive-locking element (20) with a corresponding counter element.

9. The device according to one of the preceding claims, **characterized in that** the elasticity of the support element (4) is adjustable.

10. The device according to one of the preceding claims, **characterized in that** the at least one shoulder element (2) is designed to be elastic.

11. A headrest (1), in particular for a device according to one of the preceding claims, wherein the headrest (1) has at least one shoulder element (2) and at least one support element (4) that is arranged on the at least one shoulder element (2) such that it can be detached and is elastic, the headrest (1) being designed in such a way that, when the device is mounted, it supports the user's head when the head is tilted backwards at least by a predetermined limit angle, wherein the shoulder element (2) comprises multiple brackets (6), at least one of which extends respectively across each of the two shoulders of the user.

12. The headrest (1) according to claim 11, **characterized in that** the headrest has at least one strap (32) that features at least one fastening element (34) by means of which the at least one strap (32) can be fixed to a counter bearing, which is preferably a hip strap, and/or **in that** the at least one shoulder element (2) is not made of a flexible material.

## Revendications

1. Dispositif destiné à soutenir au moins un bras d'un utilisateur, le dispositif comprenant
a. au moins un élément de soutien de bras (38) ayant un élément d'espacement et une coque pour bras destinée à être appliquée contre le bras,
b. au moins un actionneur passif (42)
i. qui est conçu pour exercer une force sur l'élément de soutien de bras (38), et
c. au moins un support antagoniste pour la force à exercer,
i. qui comprend au moins un élément de transmission de force (40) et un élément de support antagoniste,
dans lequel
l'élément d'espacement est disposé sur ledit au moins un élément de transmission de force (40) de manière à pouvoir pivoter,
le dispositif comprend un appui-tête (1) comprenant au moins un élément d'épaule (2) et au moins un élément de soutien (4) qui est disposé de manière amovible sur ledit au moins un élément d'épaule (2) et qui est élastique,
l'appui-tête (1) est conçu de telle sorte que, à l'état appliqué du dispositif, il soutient la tête de l'utilisateur lorsque la tête est inclinée vers l'arrière d'au moins un angle limite prédéterminé,
l'élément d'épaule (2) comprend plusieurs étriers (6) dont au moins un s'étend sur chacune des deux épaules de l'utilisateur.

2. Dispositif selon la revendication 1,
**caractérisé en ce que** ledit au moins un élément de soutien (4) peut être fixé audit au moins un élément d'épaule (2) dans plusieurs positions, de préférence de manière réglable en continu.

3. Dispositif selon la revendication 1 ou 2,
**caractérisé en ce qu'**au moins un élément de contact de tête (12), destiné à venir en contact contre la tête de l'utilisateur, est disposé sur ledit au moins un élément de soutien (4), qui s'adapte de préférence à un contour de la tête lorsqu'il vient en contact avec la tête de l'utilisateur.

4. Dispositif selon la revendication 3,
**caractérisé en ce que** ledit au moins un élément de contact de tête (12) présente ou est constitué d'un matériau surfacique, flexible, de préférence élastique, et est disposé sur l'élément de soutien (4) de telle sorte qu'il existe un interstice entre l'élément de contact de tête (12) et l'élément de soutien, interstice qui n'est pas fermé lors de l'utilisation conforme.

5. Dispositif selon l'une des revendications précédentes,
**caractérisé en ce que** l'appui-tête (1) comporte au moins une sangle (32), de préférence au moins deux sangles (32), qui peuvent être fixées ou sont fixées au support antagoniste, de préférence à l'élément de support antagoniste.

6. Dispositif selon la revendication 5,
**caractérisé en ce qu'**au moins une sangle (32) s'étend ventralement sur le corps de l'utilisateur, à l'état appliqué du dispositif.

7. Dispositif selon la revendication 5 ou 6,
**caractérisé en ce qu'**au moins une sangle (32) s'étend dorsalement sur le corps de l'utilisateur, à l'état appliqué du dispositif.

8. Dispositif selon l'une des revendications 5, 6 ou 7,
**caractérisé en ce qu'**au moins l'une des sangles (32) comporte un élément de complémentarité de forme (20), en particulier un élément d'encliquetage et/ou un élément de clipsage, la sangle (32) pouvant être fixée au support antagoniste par le fait que l'élément de complémentarité de forme (20) coopère avec un élément complémentaire correspondant.

9. Dispositif selon l'une des revendications précédentes,
**caractérisé en ce que** l'élasticité de l'élément de soutien (4) est réglable.

10. Dispositif selon l'une des revendications précédentes,
**caractérisé en ce que** ledit au moins un élément d'épaule (2) est réalisé de façon élastique.

11. Appui-tête (1), en particulier pour un dispositif selon l'une des revendications précédentes,
dans lequel l'appui-tête (1) comprend au moins un élément d'épaule (2) et au moins un élément de soutien (4) qui est disposé de manière amovible sur ledit au moins un élément d'épaule (2) et qui est élastique,
l'appui-tête (1) est conçu de telle sorte que, à l'état appliqué du dispositif, il soutient la tête de l'utilisateur lorsque la tête est inclinée vers l'arrière d'au moins un angle limite prédéterminé,
l'élément d'épaule (2) comprend plusieurs étriers dont au moins un s'étend sur chacune des deux épaules de l'utilisateur.

12. Appui-tête selon la revendication 11,
**caractérisé en ce que** l'appui-tête comporte au moins une sangle (32) qui présente au moins un élément de fixation (34) permettant de fixer ladite au moins une sangle (32) à un support antagoniste, de préférence à une sangle de hanche, et/ou
**en ce que** ledit au moins un élément d'épaule (2) n'est pas fabriqué en un matériau flexible.
